# EUROPEAN PATENT APPLICATION

(11) **EP 1 602 374 A1**
(43) Date of publication of application: **07.12.2005**
(21) Application number: 04717763.9
(22) Date of filing: 05.03.2004
(51) Int. Cl.: A61K 31/485, A61P 25/30, C07D 489/00

(54) **REMEDY FOR DRUG/SUBSTANCE DEPENDENCE**

(30) Priority: 07.03.2003 JP 2003061113
(71) Applicant: TORAY INDUSTRIES, INC., Tokyo 103-8666 (JP)
(72) Inventor: HASEBE, Ko, 2480034 (JP); SUZUKI, Tomohiko, 2480036 (JP); FUJII, Hideaki, 2480034 (JP); SUZUKI, Tsutomu, Yokohama-shi, Kanagawa 235-0045 (JP)
(74) Representative: Oser, Andreas
(86) International application number: PCT/JP2004/002821
(87) International publication number: WO 2004/078177

(57) **Abstract**

The present invention relates to therapeutic agents for drug/substance dependence containing a compound represented by the following compound: 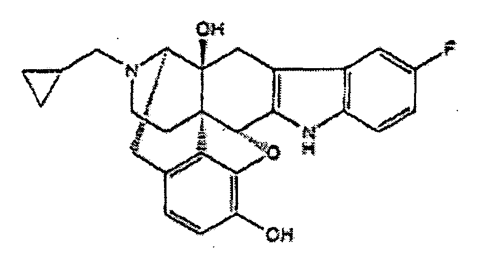 as an active ingredient. The therapeutic agents are excellent in the capability to penetrate into the brain, can promote the recovery from the state of psychologycal and physical dependence on dependence drugs/substances, can inhibit the craving and exacerbation, and are useful for the drug therapy of drug/substance dependence and drug/substance dependence patients.

## Description

### TECHNICAL FIELD

The object of the present invention is to provide therapeutic agents for drug/substance dependence. Examples of the drug/substance include dependence drugs/substances having excitatory actions on the central nervous system, particularly amphetamine type drugs (stimulant drugs), cocaine type drugs, hallucinogen (LSD) type drugs, nicotine, etc., and dependence drugs/substances having inhibitory actions on the central nervous system, particularly barbiturate and alcohol type drugs, morphine type drugs, cannabis type drugs, organic solvent type drugs, and psychotropic drugs such as benzodiazepines, and those using two or more of these drugs/substances together. The object of the present invention is to provide therapeutic agents for drug/substance dependence, which is excellent in the ability to penetrate into the brain and can inhibit the expression per se of the dependence caused by these drugs/substances, not for the conventional symptomatic therapy, in the therapy of the drug/substance dependence caused by the above-mentioned drugs/substances.

### BACKGROUND ART

If one habitually uses a natural substance such as opium, cocaine or cannabis, or a specific drug such as heroine, barbiturate or stimulant drug, or an organic solvent such as a thinner or toluene, one cannot quit using it and spends much of one's life for a mere purpose of obtaining it. Also people's habitual use of favorite tastes in daily life such as drinking (alcohols) and smoking (nicotine) also involves the same problem that the drugs/substances contained in them induce psychological or physical dependence.

The World Health Organization (WHO) defines as follows: "Drug dependence is a state, psychological and sometimes also physical, resulting from the interaction between the organism and a drug, characterized by behavioral and other responses that always include a compulsion to take a drug on a continuous or periodic basis in order to experience its psycological effects, and sometimes to avoid the discomfort of its absence." At the same time, WHO classifies the dependence-inducing drugs into eight types: (1) barbiturate and alcohol type, (2) amphetamine type, (3) cannabis type, (4) cocaine type, (5) hallucinogen (LSD) type, (6) CART type, (7) morphine type, and (8) organic solvent type. Furthermore, US National Institute on Drug Abuse (NIDA) enumerates generally abused drugs in addition to the above, such as benzodiazepines including flunitrazepam, γ-hydroxybutyrate, ketamine, phencyclidine and its derivatives, anabolic steroids, etc. Methamphetamine often abused in Japan is one of amphetamine type drugs (stimulant drugs) such as amphetamine, methylphenidate, and methylenedioxymethamphetamine, and is classified as a dependent drug/substance having an excitatory action on the central nervous system, like cocaine type drugs and nicotine. So far, neither method nor drug with a remarkable effect has been available for curing the dependence on these drugs/substances, and it is desired to develop any novel therapeutic agent.

Meanwhile, opioid receptors on which opioid acts are classified into three receptor types µ, δ and κ, and endogenous ligands and numerous synthetic ligands bound to the respective receptors are reported. It is known that agonists and antagonists for the respective types of receptors show respectively different pharmacological properties. With regard to the therapy of drug/substance dependence using opioid antagonists, known are clinical effects in the therapy of alcohol dependence by naltrexone (µ antagonist) and in the therapy of dependence on morphine type drugs such as morphine and heroine by naltrexone and buprenorphine (µ partial agonist - κ agonist). Moreover, for inhibiting the abuse or use of cocaine, a method of using naltrindole (NTI) as an opioid δ antagonist is disclosed (US Patent 5,411,965). However, on the other hand, there is also a report stating that NTI did not exert any influence on the effect of reinforcing cocaine self-administration or on the reward effect of cocaine in conditioned place preference (de Vries, T. J., et al., Psychopharmacol., 120, 442, 199), and it cannot be said that there is an established opinion concerning the effect of opioid δ antagonists on cocaine dependence. In addition, US Patent 5, 411, 965 that discloses the inhibition of cocaine use merely shows the effect of inhibiting the cocaine's reinforcing effect in the case when NTI is administered simultaneously with or before cocaine treatment, and does not show the effect of post-administration to the patients in which the state of drug/substance dependence to be usually treated has already been formed, that is, the therapeutic effect on drug/substance dependence. Furthermore, the action of NTI for inhibiting the physical dependence formed by a morphine type dependence drug is disclosed in US Patent 5, 352, 680. However, the patent merely states that NTI and its irreversible binding derivative 5'-NTII showed the effect of preventing the formation of physical dependence by morphine, and does not describe its therapeutic effect at all.

Further, as the effect of an opioid antagonist on dependence drugs/substances, a case where naloxone reduced cigarette consumption of chronic smokers is known (Karras, A., et al., Life Sci. , 27, 1541, 1980), but on the other hand, reported is a result negating the usefulness of an antagonist like naloxone as a therapeutic agent, that naloxone promoted the withdrawal symptoms of nicotine-dependent rats on the contrary while morphine as an agonist inhibited the withdrawal symptoms occurring after administration of nicotine (Malin, D. H., et al., Psychopharmacology, 112, 339, 1933). So, the opinion concerning the therapeutic effect of opioid antagonists on nicotine dependence is not yet established.

Meanwhile, the compounds of the present invention are publicly known as opioid δ ligands (PCT WO97/11948), but the gazette does not disclose the effect as therapeutic agents for drug/substance dependence at all. The present invention provides a novel medicinal application.

In general, it often occurs that a drug having its site of action in the central nervous system (in the brain) shows high activity in an *in-vitro* evaluation at the cellular level but shows little activity in an *in-vivo* evaluation made by actually administering to an animal. Several causes can be considered for the occurrence of such a phenomenon, and one of them can be the low ability of the drug to penetrate into the brain. The ability of a compound to penetrate into the brain can be an important factor for a drug expected to act in the central nervous system.

### DISCLOSURE OF THE INVENTION

The object of the present invention is to provide therapeutic agents for drug/substance dependence. Particularly the object of the present invention is to provide therapeutic agents for drug/substance dependence, which are excellent in the ability to penetrate into the brain and can inhibit the expression per se of the dependence caused by the following drugs/substances, not for the conventional symptomatic therapy, in the therapy of the drug/substance dependence caused by the drugs/substances having excitatory actions on the central nervous system, particularly amphetamine type drugs (stimulant drugs), cocaine type drugs, hallucinogen (LSD) type drugs, nicotine, etc., and dependence drugs/substances having inhibitory actions on the central nervous system, particularly barbiturate and alcohol type drugs, morphine type drugs, cannabis type drugs, organic solvent type drugs, and psychotropic drugs such as benzodiazepines, and those using two or more of these drugs/substances together.

The inventors studied intensively to solve the above-mentioned-problem, and as a result, found that the compounds represented by general formula (I) are lipophilic compounds likely to pass through the blood-brain barrier, and are especially useful for the therapy of drug/substance dependence. Thus, the present invention has been completed. The present invention relates to therapeutic agents for drug/substance dependence comprising an indole derivative represented by general formula (I) 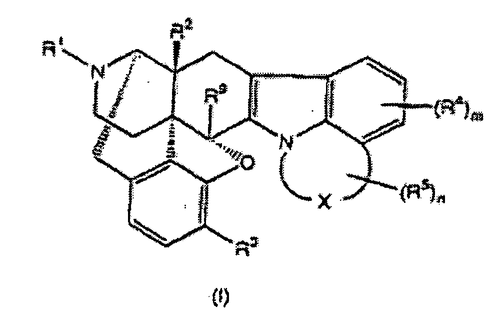 [where R¹ denotes hydrogen, alkyl with 1 to 5 carbon atoms, cycloalkylalkyl with 4 to 7 carbon atoms, cycloalkenylalkyl with 5 to 7 carbon atoms, aryl with 6 to 12 carbon atoms, aralkyl with 7 to 13 carbon atoms (where the aralkyl means an arylalkyl or arylalkenyl), alkenyl with 3 to 7 carbon atoms, furanylalkyl (the alkyl portion of which has 1 to 5 carbon atoms), or thiophenyl-alkyl (the alkyl portion of which has 1 to 5 carbon atoms);
R² denotes hydrogen, hydroxy, alkoxy with 1 to 5 carbon atoms, or alkanoyloxy with 1 to 5 carbon atoms,
R³ denotes hydrogen, hydroxy, alkoxy with 1 to 5 carbon atoms, alkanoyloxy with 1 to 5 carbon atoms, or aralkyloxy with 7 to 13 carbon atoms;
-X- denotes crosslinking consisting of 2 to 5 carbon atoms (where one or more of the carbon atoms may be substituted by a nitrogen atom, oxygen atom or sulfur atom);
m denotes an integer of 0 to 3;
n denotes an integer of 0 to 10;
m R⁴s and n R⁵s denote independently fluorine, chlorine, bromine, iodine, nitro, alkyl with 1 to 5 carbon atoms, hydroxy, alkoxy with 1 to 5 carbon atoms, trifluoromethyl, trifluoromethoxy, cyano, phenyl, isothiocyanato, SR⁶, SOR⁶, SO₂R⁶, (CH₂)ₚOR⁶, (CH₂)pCO₂R⁶, SO₂NR⁷R⁸, CONR⁷R⁸, (CH₂)ₚNR⁷R⁸, or (CH₂)ₚN(R⁷)COR⁸ (where p denotes an integer of 0 to 5; R⁶ denotes hydrogen or alkyl with 1 to 5 carbon atoms; and R⁷ and R⁸ denote, respectively independently, hydrogen, alkyl with 1 to 5 carbon atoms, or cycloalkylalkyl with 4 to 7 carbon atoms) (where among said m R⁴s and n R⁵s, two adjacent R⁴s, two adjacent R⁵s, or one R⁴ and one R⁵ adjacent to each other can be combined to form a benzene fused ring, pyridine fused ring, cyclopentane fused ring, cyclohexane fused ring, or cycloheptane fused ring);
R⁹ denotes hydrogen, alkyl with 1 to 5 carbon atoms, alkenyl with 2 to 5 carbon atoms, aralkyl with 7 to 13 carbon atoms, (CH₂)ₚOR⁶, or (CH₂)ₚCO₂R⁶ (p and R⁶ are respectively as defined before);
R¹⁰ and R¹¹ are combined to denote -O-, -S- or -CH₂-, or R¹⁰ denotes hydrogen, while R¹¹ denotes hydrogen, hydroxy, alkoxy with 1 to 5 carbon atoms, or alkanoyloxy with 1 to 5 carbon atoms], or any of its pharmacologically allowable acid addition salts as an active ingredient. The present invention also relates to a therapeutic method for drug/substance dependence using the indole derivative represented by said general formula (I) or any of its pharmacologically allowable acid addition salts, and also to the use of the indole derivative represented by said general formula (I) or any of its pharmacologically allowable acid addition salts for therapy of drug/substance dependence.

### BRIEF DESCRIPTION OF THE DRAWING

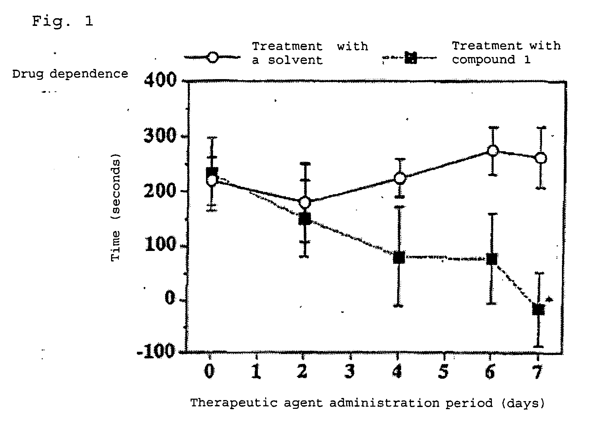

Fig. 1 shows the recovery effect of compound 1 on dependence state (therapeutic effect on stimulant drug dependence) using the reward effect in a conditioned place preference test of methamphetamine as an indicator.

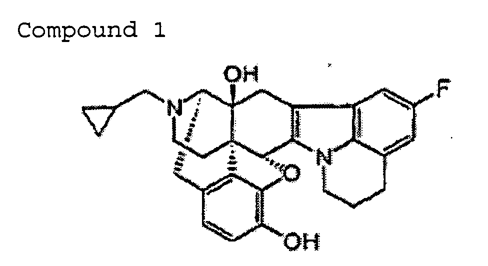

In Fig. 1, * indicates being statistically significant at a significance level of 5% or less.

### THE BEST MODES FOR CARRYING OUT THE INVENTION

As described above, the present invention relates to therapeutic agents for drug/substance dependence containing any of the compounds represented by the general formula (I) as an active ingredient.

In the compounds represented by the general formula (I), it is preferred that R¹ denotes hydrogen, alkyl with 1 to 5 carbon atoms, cycloalkylmethyl with 4 to 7 carbon atoms, cycloalkenylmethyl with 5 to 7 carbon atoms, phenyl, naphthyl, phenylalkyl with 7 to 13 carbon atoms, phenylalkenyl with 7 to 13 carbon atoms, alkenyl with 3 to 7 carbon atoms, furan-2-yl-alkyl (with 1 to 5 carbon atoms), or thiophene-2-yl-alkyl (with 1 to 5 carbon atoms). Particularly preferred is hydrogen, methyl, ethyl, propyl, butyl, cyclopropylmethyl, cyclobutylmethyl, cyclopentylmethyl, cyclohexylmethyl, cyclopentenylmethyl, cyclohexenylmethyl, benzyl, phenethyl, cinnamyl, 3-butenyl, trans-2-butenyl, prenyl, allyl, furan-2-yl-methyl, furan-2-yl-ethyl, thiophene-2-yl-methyl, or thiophene-2-yl-ethyl. Among them, especially preferred is cyclopropylmethyl, cyclobutylmethyl, cyclopentylmethyl, cyclohexylmethyl, 3-butenyl, trans-2-butenyl, prenyl, or allyl.

It is preferred that R² denotes hydrogen, hydroxy, acetoxy, propionoxy, methoxy, or ethoxy. Particularly preferred is hydrogen, hydroxy, acetoxy, or methoxy.

It is preferred that R³ denotes hydrogen, hydroxy, acetoxy, propionoxy, methoxy, ethoxy, orbenzyloxy. Particularly preferred is hydrogen, hydroxy, acetoxy, methoxy, or benzyloxy.

It is preferred that -X- denotes an alkylene with 2 to 5 carbon atoms (one carbon atom of which may also be substituted by a nitrogen atom, oxygen atom, or sulfur atom). Further preferred is an alkylene with 2 to 5 carbon atoms, -(CH₂)₂-O-, or -(CH₂)₂-S-.

It is preferred that R⁴ and R⁵ denote, respectively independently, fluorine, chlorine, bromine, iodine, nitro, alkyl with 1 to 5 carbon atoms, hydroxy, alkoxy with 1 to 5 carbon atoms, trifluoromethyl, trifluoromethoxy, cyano, phenyl, isothiocyanato, SR⁶, SOR⁶, SO₂R⁶, (CH₂)ₚOR⁶, (CH₂)ₚCO₂R⁶, SO₂NR⁷R⁸, CONR⁷R⁸, (CH₂)ₚNR⁷R⁸, or (CH₂)ₚN(R⁷)COR⁸ (where p denotes an integer of 0 to 5; R⁶ denotes hydrogen or alkyl with 1 to 5 carbon atoms; and R⁷ and R⁸ denote, respectively independently, hydrogen, alkyl with 1 to 5 carbon atoms, or cycloalkylalkyl with 4 to 7 carbon atoms). Particularly preferred is fluorine, chlorine, bromine, iodine, nitro, methyl, hydroxy, methoxy, trifluoromethyl, trifluoromethoxy, cyano, phenyl, isothiocyanato, methylthio, methylsulfinyl, methylsulfonyl, hydroxymethyl, hydroxyethyl, methoxymethyl, ethoxymethyl methoxyethyl, methoxycarbonyl, ethoxycarbonyl, methoxycarbonylmethyl, ethoxycarbonylmethyl, sulfamoyl, dimethylsulfamoyl, dimethylcarbamoyl, dimethylamino, dimethylaminomethyl, dimethylaminoethyl, or amino. Of course, a compound in which both m and n denote 0, namely, a non-substituted compound is also one of preferred compounds. Furthermore, it is preferred that two adjacent R⁴s, two adjacent R⁵s, or one R⁴ and one R⁵ adjacent to each other are combined to form at least one of benzene fused ring, pyridine fused ring, cyclopentane fused ring, cyclohexane fused ring, and cycloheptane fused ring. Especially preferred is that a benzene fused ring is formed.

It is preferred that R⁹ denotes hydrogen, alkyl with 1 to 5 carbon atoms, allyl, or benzyl. Particularly preferred is hydrogen or methyl.

It is preferred that R¹⁰ and R¹¹ are combined to denote -O-, or that R¹⁰ denotes hydrogen, while R¹¹ denotes hydrogen, hydroxy, or methoxy. It is especially preferred that both are combined to denote -O-, though R¹⁰ and R¹¹ are not limited to those enumerated here.

The compounds represented by the general formula (I) can be produced, for example, according to the method disclosed in PCT WO97/11948.

Pharmacologically preferred acid addition salts include inorganic acid salts such as hydrochlorides, sulfates, nitrates, hydrobromides, hydriodides, and phosphates, organic carboxylates such as acetates, lactates, citrates, oxalates, glutarates, malates, tartrates, fumarates, mandelates, maleates, benzoates, and phthalates, organic sulfonates such as methanesulfonates, ethanesulfonates, benzenesulfonates, p-toluenesulfonates, and camphorsulfonates, etc. Among them, preferred are hydrochlorides, hydrobromides, phosphates, tartrates, maleates, methanesulfonates, etc. The acid addition salts are not limited to those enumerated here either.

If these compounds represented by the general formula (I) pass the necessary stability test, they can be administered orally or parenterally as they are, or as medicinal compositions obtained by mixing them with any publicly known pharmacologically allowable acid, carrier, excipient, etc. Formulations employed for administering them include injections, tablets, capsules, granules, powders, syrups, suppositories, etc. The dosage can be adequately selected in response to symptom, age, body weight, administration method, etc. In the case of an injection for an adult, the dosage as an active ingredient is from 0.0001 mg to 1 g per day. In the case of an oral medicine, it is from 0.005 mg to 10 g. In either case, the dosage can be administered at a time or in several times.

Furthermore, for the purpose of enhancing the therapeutic effect for drug/substance dependence, the preparation of the present invention can further contain various adjuvants, or can also be used together with other preparations containing various adjuvants. The drugs that can be used together are not especially limited. Particular examples of them include an antipsychotic agent, antidepressant, antianxiety agent, anticonvulsant, sympathomimetic agent, NMDA receptor antagonist, calcium channel blocking agent, serotonin receptor antagonist, antihistaminic agent, opioid agent, GABA receptor function reinforcer, anti-inflammatory drug, etc. More particularly, they include clozapine, quetiapine, risperidone, haloperidol, paroxetine, fluoxetine, fluvoxamine, milnacipran, amitriptyline, imipramine, desipramine, fluoxetine, carbamazepine, diazepam, gabapentin, valproic acid, carbamazepine , clonidine, phentolamine, prazosin, ketamine, ifenprodil, mexitilene, ketanserin, sarpogrelate hydrochloride, benzodiazepine, barbiturate, fluoxetine, ondansetron, diphenhydramine, naltrexone, diclofenac, etc. Furthermore, for the therapy of alcohol dependence, disulfiram or acamprosate can be used together, and for the therapy of nicotine dependence, nicotine substitute therapy (nicotine gum, nicotine patch, or nicotine vaccine) or bupropion can also be used together. Moreover, the therapy of the present invention can also be used in combination, for example, with the electroconvulsive therapy used in the therapy for drug/substance dependence.

Examples of the drug/substance dependence to be covered by the therapy of the present invention include the drug/substance dependence caused by dependence drugs/substances having excitatory actions on the central nervous system, particularly amphetamine type drugs (stimulant drugs) such as methamphetamine, amphetamine, and methylphenidate, methylenedioxymethamphetamine, cocaine type drugs such as cocaine, hallucinogen type drugs such as LSD, nicotine, etc. and dependence drugs/substances having inhibitory actions on the central nervous system, particularly barbiturate and alcohol type drugs, morphine type drugs such as morphine, heroine, codeine, and dihydrocodeine, cannabis type drugs such as THC, organic solvent type drugs such as thinners (toluene and ethyl acetate), psychotropic agents such as benzodiazepines (triazolam, flunitrazepam, etc.), and Halcion. Furthermore, the compounds used in the present invention exhibit an effect also for the drug/substance dependence caused by two or more drugs/substances among the dependence drugs/substances.

### EXAMPLES

The present invention is particularly explained below based on examples. The following examples are described merely for exemplification, and the present invention is not limited thereto or thereby in any case.

### [Example 1]

### Evaluation of lipophilicity of compounds using a partition coefficient as an indicator

For penetration from blood to the brain, there is a barrier called the blood-brain barrier, and the lipophilicity is an important factor for the penetration into the brain. One of the parameters used for estimating the lipophilicity of a compound is partition coefficient P. The partition coefficient P is defined as the ratio of the concentration of a compound in n-octanol to the concentration of the compound in water. When the partition coefficient is larger, it expresses that the lipophilicity of the compound is higher. The partition coefficient P can be experimentally obtained and can also be obtained by calculation. For compounds 1 to 4, the logarithm of partition coefficient P, logP, values reported by Crippen, et al. (Crippen, G. M., et al., J. Chem. Inf. Comput. Sci., 27, 21, 1987) were calculated using CS Chem Draw^{R} (CambridgSoft), and the calculation results are shown in Table 1. Compounds 1 and 3 are the compounds of the present invention, and compounds 2 and 4 are comparative control compounds.

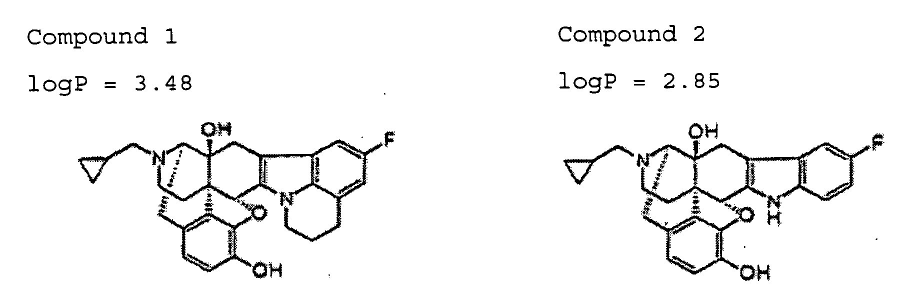

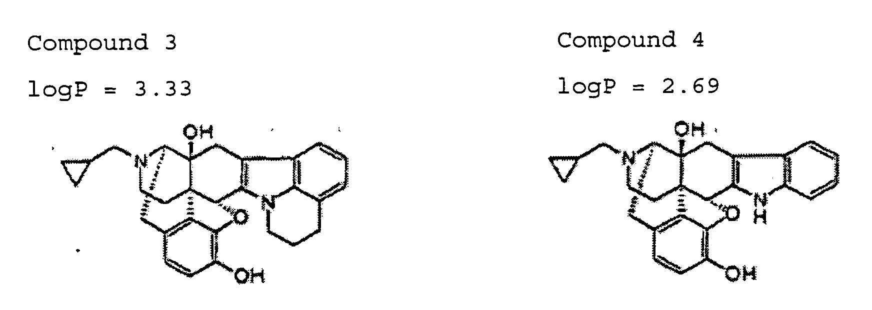

### [Example 2]

### Effect of compound 1 in promoting the recovery process after acquisition of psychological dependence by a stimulant drug (therapeutic effect for stimulant drug dependence)

The effect of compound 1 in promoting the recovery during the convalescence after the acquisition of psychological dependence by a stimulant drug was discussed using the conditioned place preference method (Suzuki, T., et al., Psychopharmacology, 102, 438, 1990: Spyraki, C., The Psychopharmacology of Addiction, p. 96, Oxford Medical Publication, New York, 1988; hereinafter called CPP method) . As a stimulant drug forming psychological dependence, methamphetamine hydrochloride was used. Furthermore, as a therapeutic agent for drug dependence, compound 1 was used.

For the experiment, SD male rats were used. The experimental apparatus used was a CPP apparatus having two compartments (one black, the other white). In the experiment, at first, the rats were trained for being conditioned with the sensory effect of the drug and the environments (white and black) in the apparatus for 6 days. The conditioned rats were placed and tested in the apparatus without drug administration. The drug dependence was evaluated with the residence time of the rats in the white and black boxes in the test session, as an indicator, in reference to whether the rats preferred the drug-induced sensory effect. As a result, the residence time in the box conditioned by the stimulant drug (2.0 mg/kg, subcutaneous) administration became long to show the formation of stimulant drug dependence (Fig. 2, 0^{th} day after start of therapy) . The rats acquiring the stimulant drug dependence obtained like this were divided into groups, and subcutaneously administered with a solvent or compound 1 (0.3 mg/kg) twice per day. On the 2^{nd}, 4^{th}, 6^{th} and 7^{th} day after start of administration of the solvent or compound 1, the rats were tested again to observe the change in the state of stimulant drug dependence.

As a result, as shown in Fig. 1, the solvent administered group maintained the state of stimulant drug dependence till 7^{th} day after start of administration. On the contrary, the group administered with compound 1 (0.3 mg/kg, subcutaneous) every day showed apparent recovery from the state of stimulant drug dependence from 4^{th} day after start of administration, and showed significant recovery on 7^{th} day after start of administration. This result shows that compound 1 exhibits an action even if it is administered after acquisition of dependence, namely, exhibits a therapeutic effect for stimulant drug dependence.

In Fig. 1, symbol * indicates being statistically significant at a significance level of 5% or less.

### INDUSTRIAL APPLICABILITY

The therapeutic agents for drug/substance dependence of the present invention contain an indole derivative represented by the general formula (I) or any of its pharmacologically allowable acid addition salts as an active ingredient and are useful for drug therapy of drug/substance dependence.
1. Therapeutic agents for drug/substance dependence comprising an indole derivative represented by general formula (I) 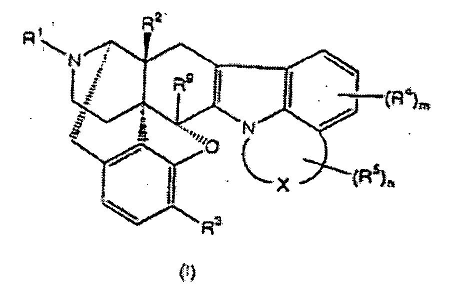 [where R¹ denotes hydrogen, alkyl with 1 to 5 carbon atoms, cycloalkylalkyl with 4 to 7 carbon atoms, cycloalkenylalkyl with 5 to 7 carbon atoms, aryl with 6 to 12 carbon atoms, aralkyl with 7 to 13 carbon atoms (where the aralkyl means an arylalkyl or arylalkenyl), alkenyl with 3 to 7 carbon atoms, furanylalkyl (the alkyl portion of which has 1 to 5 carbon atoms), or thiophenyl-alkyl (the alkyl portion of which has 1 to 5 carbon atoms);
   R² denotes hydrogen, hydroxy, alkoxy with 1 to 5 carbon atoms, alkanoyloxy with 1 to 5 carbon atoms,
   R³ denotes hydrogen, hydroxy, alkoxy with 1 to 5 carbon atoms, alkanoyloxy with 1 to 5 carbon atoms, or aralkyloxy with 7 to 13 carbon atoms;
   -X- denotes crosslinking consisting of 2 to 5 carbon atoms (where one or more of the carbon atoms may be substituted by a nitrogen atom, oxygen atom or sulfur atom);
   m denotes an integer of 0 to 3;
   n denotes an integer of 0 to 10;
   m R⁴s and n R⁵s denote, respectively independently, fluorine, chlorine, bromine, iodine, nitro, alkyl with 1 to 5 carbon atoms, hydroxy, alkoxy with 1 to 5 carbon atoms, trifluoromethyl, trifluoromethoxy, cyano, phenyl, isothiocyanato, SR⁶, SOR⁶, SO₂R⁶, (CH₂)ₚOR⁶, (CH₂)ₚCO₂R⁶, SO₂NR⁷R⁸, CONR⁷R⁸, (CH₂) ₚNR⁷R⁸, or (CH₂)ₚN(R⁷)COR⁸ (where p denotes an integer of 0 to 5; R⁶ denotes hydrogen or alkyl with 1 to 5 carbon atoms; and R⁷ and R⁸ denote, respectively independently, hydrogen, alkyl with 1 to 5 carbon atoms, or cycloalkylalkyl with 4 to 7 carbon atoms) (where among said m R⁴s and n R⁵s, two adjacent R⁴s, two adjacent R⁵s, or one R⁴ and one R⁵ adjacent to each other can be combined to form a benzene fused ring, pyridine fused ring, cyclopentane fused ring, cyclohexane fused ring, or cycloheptane fused ring);
   R⁹ denotes hydrogen, alkyl with 1 to 5 carbon atoms, alkenyl with 2 to 5 carbon atoms, aralkyl with 7 to 13 carbon atoms, (CH₂)ₚOR⁶, or (CH₂) ₚCO₂R⁶ (p and R⁶ are respectively as defined before) ;
   R¹⁰ and R¹¹ are combined to denote -O-, -S- or -CH₂-, or R¹⁰ denotes hydrogen, while R¹¹ denotes hydrogen, hydroxy, alkoxy with 1 to 5 carbon atoms, or alkanoyloxy with 1 to 5 carbon atoms], or any of its pharmacologically allowable acid addition salts as an active ingredient.
2. Therapeutic agents for drug/substance dependence, according to claim 1, which contains an indole derivative represented by the general formula (I) (where R¹⁰ and R¹¹ are combined to denote -O-) or any of its pharmacologically allowable acid addition salts, as an active ingredient.
3. Therapeutic agents for drug/substance dependence, according to claim 2, which contains an indole derivative represented by the general formula (I) (where R¹ denotes a cycloalkylalkyl with 4 to 7 carbon atoms or alkenyl with 3 to 7 carbon atoms) or any of its pharmacologically allowable acid addition salts, as an active ingredient.
4. Therapeutic agents for drug/substance dependence, according to claim 3, which contains an indole derivative represented by the general formula (I) (where R¹ denotes cyclopropylmethyl, cyclobutylmethyl, cyclopentylmethyl, cyclohexylmethyl, 3-butenyl, trans-2-butenyl, prenyl, or allyl; R² denotes hydrogen, hydroxy, acetoxy, or methoxy; R³ denotes hydrogen, hydroxy, acetoxy, methoxy, or benzyloxy; -X- denotes an alkylene with 2 to 5 carbon atoms, -(CH₂)₂-O-, or -(CH₂)₂-S-; m and n denote independently 0 or 1; R⁴ and R⁵ denote, respectively independently, fluorine, chlorine, bromine, iodine, nitro, methyl, hydroxy, methoxy, trifluoromethyl, trifluoromethoxy, cyano, phenyl, isothiocyanato, methylthio, methylsulfinyl, methylsulfonyl, hydroxymethyl, hydroxyethyl, methoxymethyl, ethoxymethyl, methoxyethyl, methoxycarbonyl, ethoxycarbonyl, methoxycarbonylmethyl, ethoxycarbonylmethyl, sulfamoyl, dimethylsulfamoyl, dimethylcarbamoyl, dimethylamino, dimethylaminomethyl, dimethylaminoethyl, or amino; and R⁹ denotes hydrogen or methyl) or any of its pharmacologically allowable acid addition salts, as an active ingredient.
5. Therapeutic agents for drug/substance dependence, according to any one of claims 1 through 4, wherein the drug/substance dependence is dependence caused by a drug/substance having an excitatory action on the central nervous system.
6. Therapeutic agents for drug/substance dependence, according to any one of claims 1 through 4, wherein the drug/substance dependence is dependence caused by a nicotine or amphetamine type drug (stimulant drug).
7. Therapeutic agents for drug/substance dependence, according to any one of claims 1 through 4, wherein the drug/substance dependence is dependence caused by a cocaine type drug.
8. Therapeutic agents for drug/substance dependence, according to any one of claims 1 through 4, wherein the drug/substance dependence is dependence caused by a dependence drug/substance having an inhibitory action on the central nervous system.
9. A therapeutic method for drug/substance dependence using the indole derivative or any of its pharmacologically allowable acid addition salts as set forth in any one of claims 1 through 4.
10. Use of the indole derivative or any of its pharmacologically allowable acid addition salts as set forth in any one of claims 1 through 4, for the therapy of drug/substance dependence.

## Claims

1. Therapeutic agents for drug/substance dependence comprising an indole derivative represented by general formula (I) 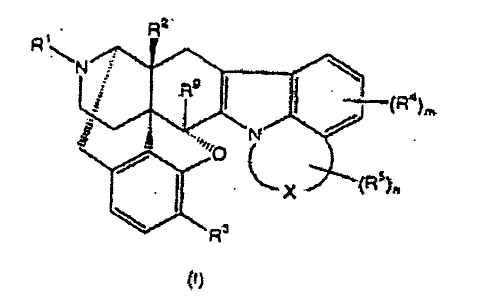 [where R¹ denotes hydrogen, alkyl with 1 to 5 carbon atoms, cycloalkylalkyl with 4 to 7 carbon atoms, cycloalkenylalkyl with 5 to 7 carbon atoms, aryl with 6 to 12 carbon atoms, aralkyl with 7 to 13 carbon atoms (where the aralkyl means an arylalkyl or arylalkenyl), alkenyl with 3 to 7 carbon atoms, furanylalkyl (the alkyl portion of which has 1 to 5 carbon atoms), or thiophenyl-alkyl (the alkyl portion of which has 1 to 5 carbon atoms);
R² denotes hydrogen, hydroxy, alkoxy with 1 to 5 carbon atoms, alkanoyloxy with 1 to 5 carbon atoms,
R³ denotes hydrogen, hydroxy, alkoxy with 1 to 5 carbon atoms, alkanoyloxy with 1 to 5 carbon atoms, or aralkyloxy with 7 to 13 carbon atoms;
-X- denotes crosslinking consisting of 2 to 5 carbon atoms (where one or more of the carbon atoms may be substituted by a nitrogen atom, oxygen atom or sulfur atom);
m denotes an integer of 0 to 3;
n denotes an integer of 0 to 10;
m R⁴s and n R⁵s denote, respectively independently, fluorine, chlorine, bromine, iodine, nitro, alkyl with 1 to 5 carbon atoms, hydroxy, alkoxy with 1 to 5 carbon atoms, trifluoromethyl, trifluoromethoxy, cyano, phenyl, isothiocyanato, SR⁶, SOR⁶, SO₂R⁶, (CH₂)ₚOR⁶, (CH₂)ₚCO₂R⁶, SO₂NR⁷R⁸, CONR⁷R⁸, (CH₂)ₚNR⁷R⁸, or (CH₂)ₚN(R⁷)COR⁸ (where p denotes an integer of 0 to 5; R⁶ denotes hydrogen or alkyl with 1 to 5 carbon atoms; and R⁷ and R⁸ denote, respectively independently, hydrogen, alkyl with 1 to 5 carbon atoms, or cycloalkylalkyl with 4 to 7 carbon atoms) (where among said m R⁴s and n R⁵s, two adjacent R⁴s , two adjacent R⁵s , or one R⁴ and one R⁵ adjacent to each other can be combined to form a benzene fused ring, pyridine fused ring, cyclopentane fused ring, cyclohexane fused ring, or cycloheptane fused ring);
R⁹ denotes hydrogen, alkyl with 1 to 5 carbon atoms, alkenyl with 2 to 5 carbon atoms, aralkyl with 7 to 13 carbon atoms, (CH₂)ₚOR⁶, or (CH₂)pCO₂R⁶ (p and R⁶ are respectively as defined before)];
or any of its pharmacologically allowable acid addition salts as an active ingredient.

2. Therapeutic agents for drug/substance dependence, according to claim 1, which contains an indole derivative represented by the general formula (I) (where R¹ denotes a cycloalkylalkyl with 4 to 7 carbon atoms or alkenyl with 3 to 7 carbon atoms) or any of its pharmacologically allowable acid addition salts, as an active ingredient.

3. Therapeutic agents for drug/substance dependence, according to claim 1 or 2, which contains an indole derivative represented by the general formula (I) (where R¹ denotes cyclopropylmethyl, cyclobutylmethyl, cyclopentylmethyl, cyclohexylmethyl, 3-butenyl, trans-2-butenyl, prenyl, or allyl; R² denotes hydrogen, hydroxy, acetoxy, or methoxy; R³ denotes hydrogen, hydroxy, acetoxy, methoxy, or benzyloxy; -X- denotes an alkylene with 2 to 5 carbon atoms, -(CH₂)₂-O-, or -(CH₂)₂-S-; m and n denote independently 0 or 1; R⁴ and R⁵ denote, respectively independently, fluorine, chlorine, bromine, iodine, nitro, methyl, hydroxy, methoxy, trifluoromethyl, trifluoromethoxy, cyano, phenyl, isothiocyanato, methylthio, methylsulfinyl, methylsulfonyl, hydroxymethyl, hydroxyethyl, methoxymethyl, ethoxymethyl, methoxyethyl, methoxycarbonyl, ethoxycarbonyl, methoxycarbonylmethyl, ethoxycarbonylmethyl, sulfamoyl, dimethylsulfamoyl, dimethylcarbamoyl, dimethylamino, dimethylaminomethyl, dimethylaminoethyl, or amino; and R⁹ denotes hydrogen or methyl) or any of its pharmacologically allowable acid addition salts, as an active ingredient.

4. Therapeutic agents for drug/substance dependence, according to any one of claims 1 through 3, wherein the drug/substance dependence is dependence caused by a drug/substance having an excitatory action on the central nervous system.

5. Therapeutic agents for drug/substance dependence, according to any one of claims 1 through 3, wherein the drug/substance dependence is dependence caused by a nicotine or amphetamine type drug (stimulant drug).

6. Therapeutic agents for drug/substance dependence, according to any one of claims 1 through 3, wherein the drug/substance dependence is dependence caused by a cocaine type drug.

7. Therapeutic agents for drug/substance dependence, according to any one of claims 1 through 3, wherein the drug/substance dependence is dependence caused by a dependence drug/substance having an inhibitory action on the central nervous system.

8. Use of the indole derivative or any of its pharmacologically allowable acid addition salts as set forth in any one of claims 1 through 3, for the preparation of a therapeutic agent for the therapy of drug/substance dependence.
